# EUROPEAN PATENT SPECIFICATION

(11) **EP 3 039 351 B1**
(45) Date of publication and mention of the grant of the patent: **15.08.2018**
(21) Application number: 14840740.6
(22) Date of filing: 29.08.2014
(51) Int. Cl.: F24F 3/14, F24F 6/14, H01R 13/15, H01R 13/24, A61L 9/14, B05B 5/057, B05B 5/053, F24F 3/16, B05B 7/00

(54) **ELECTROSTATIC SPRAY MODULE AND AIR CONDITIONER HAVING THE SAME**
ELEKTROSTATISCHE SPRÜHEINHEIT UND KLIMAANLAGE DAMIT
MODULE DE PULVÉRISATION ÉLECTROSTATIQUE ET APPAREIL DE CONDITIONNEMENT D'AIR POSSÉDANT CELUI-CI

(30) Priority: 30.08.2013 KR 20130103872
(43) Date of publication of application: 06.07.2016
(73) Proprietor: LG Electronics Inc., Seoul 07336 (KR)
(72) Inventor: CHOI, Ji Eun, Changwon-si Gyeongsangnam-do 642-711 (KR); KIM, Woo Jin, Changwon-si Gyeongsangnam-do 642-711 (KR); KIM, Ho Jung, Changwon-si Gyeongsangnam-do 642-711 (KR)
(74) Representative: Ter Meer Steinmeister & Partner
(86) International application number: PCT/KR2014/008105
(87) International publication number: WO 2015/030538

(56) References cited:
- EP-A1- 0 503 766
- EP-A1- 1 629 897
- KR-A- 20010 104 026
- KR-A- 20070 019 335
- KR-Y1- 200 302 929
- US-A- 5 805 768
- US-A- 5 957 771
- US-A1- 2012 207 651

## Description

### Technical Field

The present invention relates to an electrostatic spray module and an air conditioner having the same, particularly an electrostatic spray module including an electrostatic spray capsule holder to/from which an electrostatic spray capsule is attached/detached, and an air conditioner having the electrostatic spray module.

### Background Art

In general, electrostatic spray devices, which are spray devices that can spray liquid, are devices spraying liquid by applying high voltage to an electrode and a charged electrode.

The electrostatic spray devices may include a container keeping liquid, an electrode sucking liquid from the container, a ground electrode for generating a potential difference from the electrode, and a high-voltage power applying high voltage to between the electrode and the ground electrode.

In the electrostatic spray devices, liquid can be absorbed to the electrode by the capillary phenomenon, and when high voltage is applied from the high-voltage power, an electric force larger than the surface tension of the liquid is generated and the drops of the liquid are atomized and split into mist by the potential difference between the electrode and the ground electrode, such that the liquid can be sprayed.

EP 1 629 897 A1 describes a liquid stored in the liquid storing means within a housing is supplied to a carrier. A high voltage applied to a discharge end of the carrier and an opposed electrode to emit tiny ionized liquid particles. At least part of the liquid storing means is detachable to the housing for easy replenishment of the liquid.

### Disclosure of Invention

### Technical Problem

An object of the present invention is to provide an electrostatic spray module capable of conveniently serve an electrostatic spray capsule, and an air conditioner having the electrostatic spray module.

### Solution to Problem

The present invention includes: an electrostatic spray capsule with terminals; a high voltage generator; an electrostatic spray capsule holder where the electrostatic spray capsule is attached/detached; and a conducting mechanism mounted on the electrostatic spray capsule holder, connected with the high voltage generator, and coming in contact with the terminals by partially elastically deforming, when the electrostatic spray capsule is mounted.

The conducting mechanism includes: a connection terminal connected with the high voltage generator; a contact member coming in contact with the terminal, when the electrostatic spray capsule is mounted; and an elastic member elastically supporting the contact member toward the terminal.

The connection terminal may be connected with the elastic member.

The contact member may come in contact with the connection terminal, when the electrostatic spray capsule is mounted.

The electrostatic spray module further includes: a spring disposed in the electrostatic spray capsule holder; and an elastic holder having balls that are supported by the spring and brought in contact with the electrostatic spray capsule, when the electrostatic spray capsule is mounted.

The terminal may include a ground terminal with a portion exposed to the outside of the electrostatic spray capsule and a high voltage applying terminal with a portion exposed to the outside of the electrostatic spray capsule. The conducting mechanism may include a ground terminal conducting mechanism electrically connected with the ground terminal and a high voltage applying terminal conducting mechanism electrically connected with the high voltage applying terminal.

The electrostatic spray module further includes: a spring disposed in the electrostatic spray capsule holder; and an elastic holder having balls that are supported by the spring and brought in contact with the electrostatic spray capsule, when the electrostatic spray capsule is mounted. One of a contact member of the ground terminal conducting mechanism and a contact member of the high voltage applying terminal conducting mechanism may be disposed to face the balls.

The present invention may include: an electrostatic spray capsule disposed such that a portion of a ground terminal and a portion of a high voltage applying terminal are exposed to the outside; a high voltage generator; an electrostatic spray capsule holder where the electrostatic spray capsule is attached/detached; a ground terminal conducting mechanism mounted on the electrostatic spray capsule holder, connected with the high voltage generator, and coming in contact with the ground terminal by partially elastically deforming, when the electrostatic spray capsule is mounted; and a high voltage applying terminal conducting mechanism mounted on the electrostatic spray capsule holder, connected with the high voltage generator, and coming in contact with the high voltage applying terminal by partially elastically deforming, when the electrostatic spray capsule is mounted.

The ground terminal conducting mechanism may include: a connection terminal connected with the high voltage generator; a contact member coming in contact with the ground terminal, when the electrostatic spray capsule is mounted; and an elastic member elastically supporting the contact member toward the electrostatic spray capsule.

The high voltage applying terminal conducting mechanism may include: a connection terminal connected with the high voltage generator; a contact member coming in contact with the high voltage applying terminal, when the electrostatic spray capsule is mounted; and an elastic member elastically supporting the contact member toward the electrostatic spray capsule.

The electrostatic spray module further includes: a spring disposed in the electrostatic spray capsule holder; and an elastic holder having balls that are supported by the spring and brought in contact with the electrostatic spray capsule, when the electrostatic spray capsule is mounted.

The balls may be disposed to face one of the ground terminal conducting mechanism and the high voltage applying terminal conducting mechanism.

The present invention may include: a body that has an air intake unit, an air discharge unit, and an air channel between the air intake unit and the air discharge unit; a heat exchanger disposed in the air channel; a blowing unit that takes air from the air intake unit and sends the air to the air discharge unit through the heat exchanger; and an electrostatic spray module disposed in the body, in which the electrostatic spray module includes: an electrostatic spray capsule with a terminal; a high voltage generator; an electrostatic spray capsule holder where the electrostatic spray capsule is attached/ detached; and a conducting mechanism mounted on the electrostatic spray capsule holder, connected with the high voltage generator, and coming in contact with the terminal by partially elastically deforming, when the electrostatic spray capsule is mounted.

The conducting mechanism includes: a connecting terminal connected with the high voltage generator; a contact member coming in contact with the terminal, when the electrostatic spray capsule is mounted; and an elastic member elastically supporting the contact member toward the terminal.

The electrostatic spray module may include a housing having the electrostatic spray capsule holder and an entrance at a side through which the electrostatic spray capsule is put inside/outside.

The high voltage generator may be disposed in the housing.

The air discharge unit may include a discharge body having an open air discharge space through which air is discharge to an interior. The discharge body may have a module seat in which the electrostatic spray module is received. The electrostatic spray module may spray a functional substance to the air discharge space.

The electrostatic spray module further includes: a spring disposed in the electrostatic spray capsule holder; and an elastic holder having balls that are supported by the spring and brought in contact with the electrostatic spray capsule, when the electrostatic spray capsule is mounted.

### Advantageous Effects of Invention

According to the present invention, it is possible to mount the electrostatic spray capsule with high reliability and provide convenient service for the electrostatic spray capsule.

Further, since the electrostatic spray capsule comes in contact with the conducting mechanism while elastically deforming, when the electrostatic spray capsule is mounted, it is easy to attach/detach the electrostatic spray capsule.

Further, it is possible to conveniently attach/detach the electrostatic spray capsule without disassembling an air conditioner.

### Brief Description of Drawings

FIG. 1 is a perspective view showing an embodiment of an electrostatic spray module according to the present invention.
FIG. 2 is a perspective view when an electrostatic spray capsule of an embodiment of an electrostatic spray module according to the present invention is separated to the outside.
FIG. 3 is a cross-sectional view showing a ground terminal of the electrostatic spray capsule shown in FIG. 2.
FIG. 4 is a cross-sectional view showing a voltage applying terminal of the electrostatic spray capsule shown in FIG. 2.
FIG. 5 is a perspective view showing an electrostatic spray capsule holder of an embodiment of an electrostatic spray module according to the present invention.
FIG. 6 is a cross-sectional view when the ground terminal of an embodiment of an electrostatic spray module according to the present invention is brought in contact with a conducting mechanism of the electrostatic spray capsule holder.
FIG. 7 is a cross-sectional view when the electrostatic spray capsule shown in FIG. 6 is separated from the electrostatic spray capsule holder.
FIG. 8 is a cross-sectional view when a high voltage applying terminal of an embodiment of an electrostatic spray module according to the present invention is brought in contact with a conducting mechanism of the electrostatic spray capsule holder.
FIG. 9 is a cross-sectional view when the electrostatic spray capsule shown in FIG. 8 is separated from the electrostatic spray capsule holder.
FIG. 10 is a front view of an embodiment of an air conditioner equipped with an electrostatic spray module according to an embodiment of the present invention.
FIG. 11 is a cross-sectional view of an example of an air conditioner equipped with an electrostatic spray module according to an embodiment of the present invention.
FIG. 12 is a cross-sectional view when an electrostatic spray capsule is separated in an embodiment of an air conditioner equipped with an electrostatic spray module according to the present invention.

### Best Mode for Carrying out the Invention

Hereinafter, embodiments of the present invention will be described in detail with reference to the accompanying drawings.

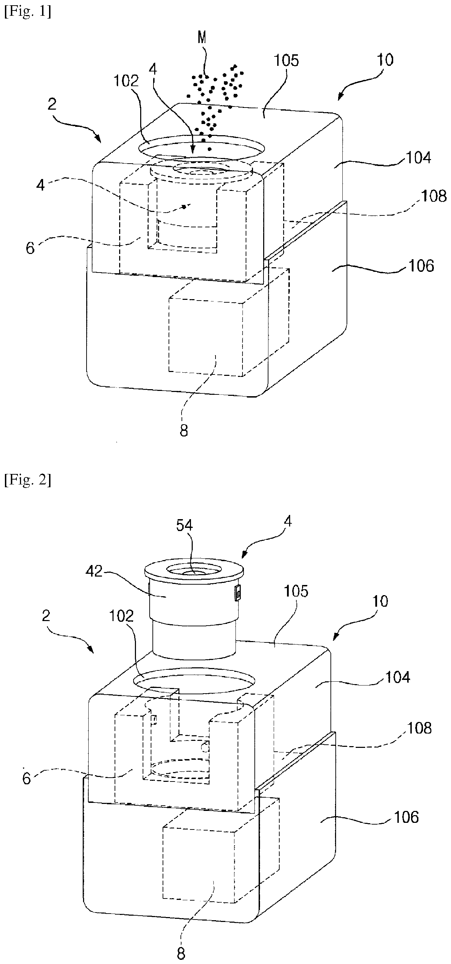

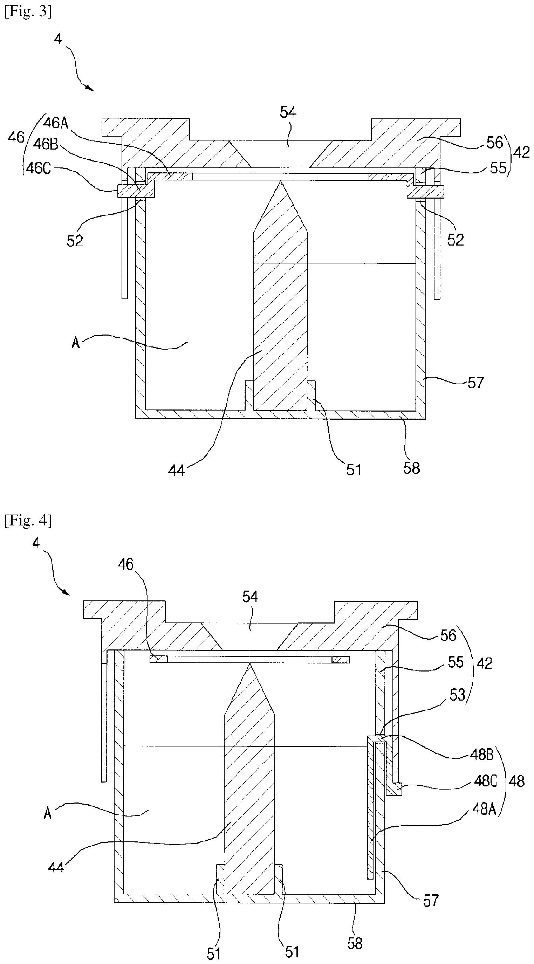

FIG. 1 is a perspective view showing an embodiment of an electrostatic spray module according to the present invention, FIG. 2 is a perspective view when an electrostatic spray capsule of an embodiment of an electrostatic spray module according to the present invention is separated to the outside, FIG. 3 is a cross-sectional view showing a ground terminal of the electrostatic spray capsule shown in FIG. 2, and FIG. 4 is a cross-sectional view showing a voltage applying terminal of the electrostatic spray capsule shown in FIG. 2.

An electrostatic spray module 2 includes an electrostatic spray capsule 4, and electrostatic spray capsule holder 6, and a high voltage generator 8.

The electrostatic spray capsule 4 can keep a functional substance A in a container 42, and the functional substance A can be atomized and sprayed to the outside, when high voltage is applied from the outside. The electrostatic spray capsule 4 is a part that sprays the functional substance A by generating a potential difference by charging fluid to the functional substance A. The functional substance A may be an aroma oil such as a rosemary aroma oil, a peppermint aroma oil, a lemon aroma oil, and a basil aroma oil. The functional substance A can be put in the container 42 in a liquid state and its drops can be atomized and sprayed by an electric force generated by an applied high voltage. The electrostatic spray capsule 4 may include the container 42 in which the functional substance A is put and a porous electrode 44 that is disposed in the container 42 and through which the functional substance A is moved up by the capillary phenomenon. The electrostatic spray capsule 4 has a terminal. The terminal may include a ground terminal 46 generating a potential difference from the porous electrode 44 and a high voltage applying terminal 48 applying high voltage generated by the high voltage generator 8 to the functional substance A.

An electrode supporter 51 supporting the porous electrode 44 may be formed in the container 42. A ground terminal hole 52 through which the ground terminal 46 passes may be formed at the container 42. A high voltage applying terminal hole 53 through which the high voltage applying terminal 48 passes may be formed at the container 42. A discharge hole 54 through which a mist produced in the container 2 is discharged may be formed at the container 2.

The container 42 may include a body 55 of which the top is open and which has a space for keeping the functional substance A therein, and a cover 56 covering the top of the body 55. The body 55 may include a hollow tub 57 and a bottom plate 58 blocking the bottom of the hollow tub 57. In the container 42, the electrode supporter 51, the ground terminal hole 52, and the high voltage applying terminal hole 53 may be formed at the body 55 and the discharge hole 54 may be formed at the cover 56.

The electrode supporter 51 can be formed in the container 42 to support the lower portion of the porous electrode 44. The electrode supporter 51 may protrude integrally from the container 42, in the container 42. The electrode supporter 51 may be formed on the body 55. The electrode supporter 51 may protrude upward on the upper surface of the bottom plate 58. A plurality of electrode supporters 51 may be formed and spaced from each other. A plurality of electrode supporters 51 may be formed around a portion of the porous electrode 44. The functional substance A can flow to the porous electrode 44 through the gaps between the electrode supporters 51.

The ground terminal hole 52 may be formed at the hollow tub 57, at a predetermined distance from the high voltage applying terminal hole 53. At least one ground terminal hole 52 may be formed at the hollow tub 57 and two may be formed to face each other.

The high voltage applying terminal hole 53 may be formed at the hollow tub 57. The high voltage applying terminal hole 53 and the ground terminal hole 52 may be formed at different heights on the hollow tub 57. The height of the high voltage applying terminal hole 53 from the bottom plate 58 may be smaller than the height of the ground terminal hole 52 from the bottom plate 58.

The discharge hole 54 may be formed such that the open area of the top is larger than the open area of the bottom. The discharge hole 54 may be formed such that the area gradually increases, as it goes to the top. The discharge hole 54 may be formed in a cone shape. The discharge hole 14 may enable the functional substance A to easily flow into the container 42, when the functional substance A flows inside from the outside. The cover 56 may be formed in a cylinder shape with the bottom open. The discharge hole 54 may be formed in the cover 56 and may be positioned over the porous electrode 44. The discharge hole 54 may be sized such that the portion of the ground terminal 46 which is positioned inside the container 42 is not seen through the discharge hole 54, when the body 55 is covered with the cover 56.

The porous electrode 44 may be disposed inside the container 42. In the porous electrode 44, the lower portion may be fixed to the container 42 and the upper portion may be disposed close to the ground terminal 46. The electrostatic spray device sprays the functional substance, using a potential difference between the porous electrode 44 and the ground terminal 46 and the porous electrode 44 may be made of a nonconductive material such as polyester, acryl, and carbon fiber. The liquid-state functional substance A in the container 42 may be moved to the upper portion of the porous electrode 44 by the capillary phenomenon. Electrostatic spray has various spray modes, but cone-jet mode spray having a cone shape is the most stable. On the other hand, when the functional substance A is nonpolar fluid, it is difficult to form cone-jet due to low electric conductivity, so stable spray of nonpolar fluid can be induced by artificially making the top in a cone type. The porous electrode 4 may be formed in a needle shape vertically long and the upper portion of the porous electrode 44 may be pointed in a cone shape. That is, the porous electrode 44 may have a pointed top. The porous electrode 44 can generate a strong electric field at the pointed top, when high voltage is applied, and monopole charges produced in this case permeate into the functional substance A, thereby facilitating monopole charging.

The ground terminal 46 may be disposed to be partially exposed to the outside of the electrostatic spray capsule 4. The ground terminal 46, as shown in FIG. 3, may include an inner terminal portion 46A disposed in the container 42, a container through-portion 46B passing through the container 42, and an outer terminal portion 46C disposed outside the container 42. The ground terminal 46 may be disposed such that the outer terminal portion 46C is exposed to the upper outside of the container 42.

The high voltage applying terminal 48 may be disposed to be partially exposed to the outside of the electrostatic spray capsule 4. The high voltage applying terminal 48, as shown in FIG. 4, may further include an inner terminal portion 48A disposed in the container 2, a container through-portion 48B passing through the container, and an outer terminal portion 48C disposed outside the container 42. The high voltage applying terminal 48 may be disposed such that the outer terminal portion 48C is exposed to the lower outside of the container 42.

The electrostatic spray capsule 4 can be mounted on the electrostatic spray capsule holder 6. The electrostatic spray capsule 4 is detachably mounted on the electrostatic spray capsule holder 6. The electrostatic spray capsule 4 can spray a mist M to the outside, on the electrostatic spray capsule holder 6. The electrostatic spray capsule 4 can be in contact with a conducting mechanism and electrically connected with the high voltage generator 8 through the conducting mechanism, when mounted on the electrostatic spray capsule holder 6. The electrostatic spray capsule holder 6 is important in the contact between the conducting mechanism and the electrostatic spray capsule 4, when the electrostatic spray capsule 4 is mounted, and it is preferable that the conducting mechanism has a structure allowing easy attachment/detachment of the electrostatic spray capsule 4 and also capable of ensuring contact with the electrostatic spray capsule 4. The conducting mechanism will be described below.

The electrostatic spray capsule holder 6 may be formed such that two sides facing each other in four of the front, rear, left, and right sides are closed and an opening is formed at at least one side of the other sides. The left side and the right side of the electrostatic spray capsule holder 6 may be closed, and an opening may be formed at the front side of the electrostatic spray capsule holder 6 and the rear side of the electrostatic spray capsule holder 6. In contrast, the front side and the rear side of the electrostatic spray capsule holder 6 may be closed, and an opening may be formed at the left side of the electrostatic spray capsule holder 6 and the right side of the electrostatic spray capsule holder 6. A user or a worker (hereafter, referred to as a user) can put fingers into the openings to attach/detach the electrostatic spray capsule 4 and can easily attach/detach the electrostatic spray capsule 4 to/from the electrostatic spray capsule holder 6.

The high voltage generator 8 may be connected with the conducting mechanism by a lead wire. The conducting mechanism may be mounted on the electrostatic spray capsule holder 6 and connected to the high voltage generator 8. The conducting mechanism comes in contact with a terminal on the electrostatic spray capsule 4, when the electrostatic spray capsule 4 is mounted. It is preferable that the high voltage generator 8 generates high voltage of about 6 ~ 8 kv and applies it to the conducting mechanism, for stable spraying.

The electrostatic spray module 2 may include a housing 10 forming the external appearance of the electrostatic spray module 2. An entrance 102 through which the electrostatic spray capsule 4 is put inside/outside may be formed at the housing 10. The entrance 102 may be disposed over the discharge hole 54 of the electrostatic spray capsule 4, when the electrostatic spray capsule 4 is mounted on the electrostatic spray capsule holder 6. The electrostatic spray capsule holder 6 and the high voltage generator 8 may be disposed in the housing 10. The housing 10 may be formed separately from the electrostatic spray capsule holder 6 and then combined with the electrostatic spray capsule holder 6 or may be formed integrally with the electrostatic spray capsule holder 6. The electrostatic spray capsule 4 can be mounted on the electrostatic spray capsule holder 6 through the entrance 102 and can be put out of the housing 10 through the entrance 102. The entrance 102 can function as an electrostatic spray capsule path through which the electrostatic spray capsule 4 is put in/out of the housing 10 and can function as a mist outlet through which the mist M sprayed from the electrostatic spray capsule 4 can flow out of the electrostatic spray module 2.

The housing 10 may include an upper housing 104 protecting the electrostatic spray capsule holder 6 and a lower housing 106 protecting the high voltage generator 8.

The upper housing 104 may be formed larger than the electrostatic spray capsule holder 6. The upper housing 104 may have the entrance 102 at the top 105. The upper housing 104 may be disposed on the lower housing 106. The upper housing 104 may be combined with the lower housing 106 by locking members such as hooks or by fasteners such as screws. The entrance 102 may be vertically open through the top 123 of the upper housing 104. The entrance 102 may be formed smaller in area than the electrostatic spray capsule holder 6 and larger in area than the electrostatic spray capsule 4. The electrostatic spray capsule 4 can be inserted into the electrostatic spray capsule holder 6 through the entrance 102 from above the entrance 102 and seated in the electrostatic spray capsule holder 6. When the electrostatic spray capsule holder 6 is formed integrally with the housing 10, the electrostatic spray capsule holder 6 may be formed integrally with the upper housing 104.

The lower housing 106 may be formed larger than the high voltage generator 140. The housing 10 may further include a separator 108 dividing its inside up and down. The electrostatic spray capsule holder 6 may be disposed on the separator 108 and the high voltage generator 140 may be disposed under the separator 108.

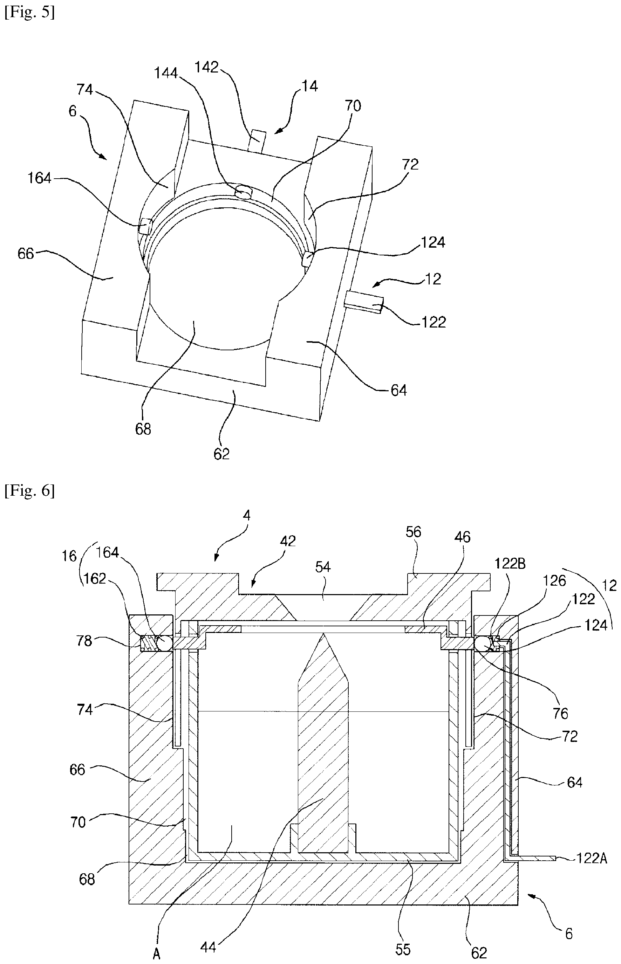

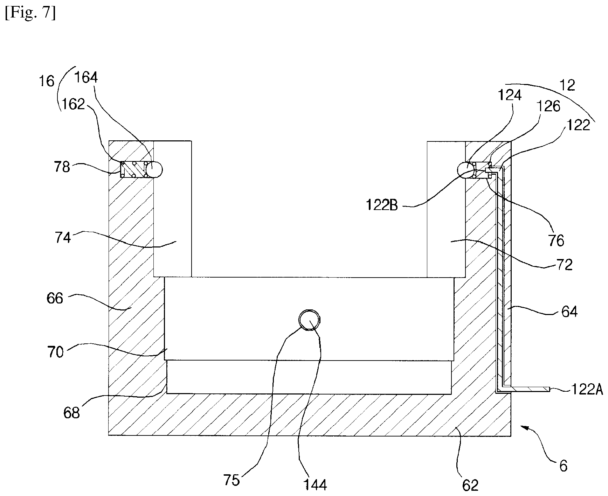

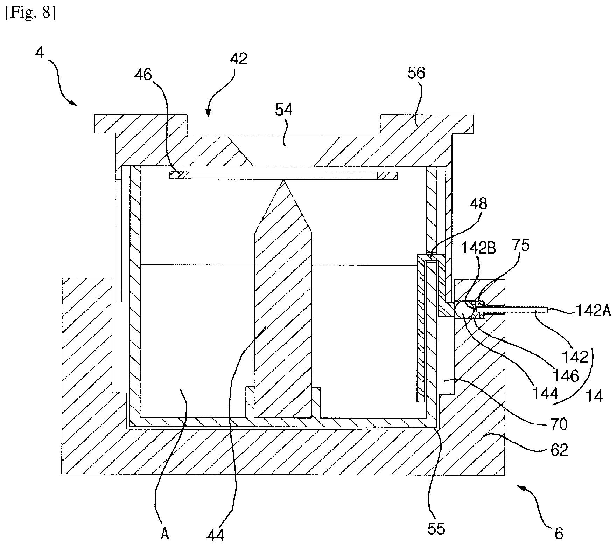

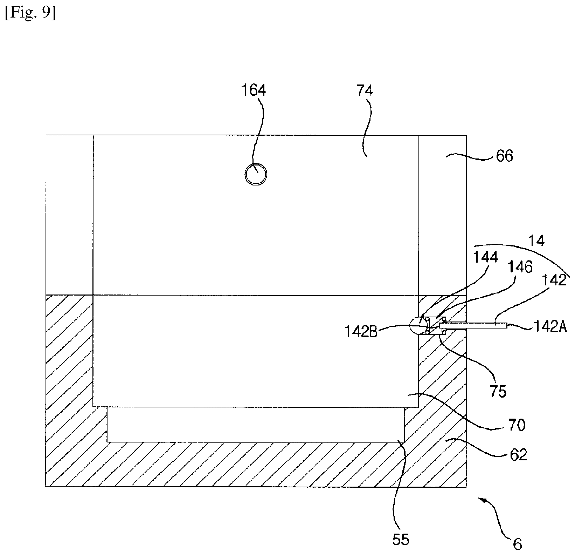

FIG. 5 is a perspective view showing an electrostatic spray capsule holder of an embodiment of an electrostatic spray module according to the present invention, FIG. 6 is a cross-sectional view when the ground terminal of an embodiment of an electrostatic spray module according to the present invention is brought in contact with a conducting mechanism of the electrostatic spray capsule holder, FIG. 7 is a cross-sectional view when the electrostatic spray capsule shown in FIG. 6 is separated from the electrostatic spray capsule holder, FIG. 8 is a cross-sectional view when a high voltage applying terminal of an embodiment of an electrostatic spray module according to the present invention is brought in contact with a conducting mechanism of the electrostatic spray capsule holder, and FIG. 9 is a cross-sectional view when the electrostatic spray capsule shown in FIG. 8 is separated from the electrostatic spray capsule holder.

The conducting mechanisms 12 and 14 are mounted on the electrostatic spray capsule holder 6 and connected to the high voltage generator 8. The conducting mechanisms 12 and 14 can be brought in contact with the terminals 46 and 48 by partially elastically deforming, when the electrostatic spray capsule 4 is mounted.

The conducting mechanisms 12 and 14 may include connection terminals 122 and 142 connected with the high voltage generator 8, contact members 124 and 144 brought in contact with the terminals 46 and 48, when the electrostatic spray capsule 4 is mounted, and elastic members 126 and 146 being in contact with the contact members 124 and 144 and elastically supporting the contact members 124 and 144 toward the terminals 46 and 48.

The conducting mechanisms 12 and 14 can keep the connection terminals 122 and 142 connected with the elastic members 126 and 146 and the elastic members 126 and 146 can electrically connect the contact members 124 and 144 and the connection terminals 122 and 142 to each other.

In the conducting mechanisms 12 and 14, the contact members 124 and 144 can come in contact with the connection terminals 122 and 142, when the electrostatic spray capsule 4 is mounted. In the conducting mechanisms 12 and 14, the contact members 124 and 144 can pushed to the connection terminals 122 and 142 by the electrostatic spray capsule 4, when the electrostatic spray capsule 4 is mounted. The contact members 124 and 144 and the connection members 122 and 142 may come in direct contact with each other. The contact members 124 and 144 may be pushed opposite to the connection terminals 122 and 142 by the elastic members 126 and 146, when the electrostatic spray capsule 4 is separated, in which the contact members 124 and 144 and the connection terminals 122 and 142 cannot be brought in contact with each other.

The conducting mechanisms 12 and 14 may include a ground terminal conducting mechanism 12 that is electrically connected with the ground terminal 46 and a high voltage applying terminal conducting mechanism 14 that is electrically connected with the high voltage applying terminal 48.

Hereinafter, the ground terminal conducting mechanism 12 and high voltage applying terminal conducting mechanism 14 are described individually, when there is a need of discriminating the ground terminal conducting mechanism 12 and high voltage applying terminal conducting mechanism 14, but the 'term conducting mechanisms 12 and 14' is used for the common configuration of the ground terminal conducting mechanism 12 and high voltage applying terminal conducting mechanism 14.

The conducting mechanisms 12 and 14 can come in contact with the terminal of the electrostatic spray capsule 4 and elastically support the electrostatic spray capsule 4 by elastically deforming, when the electrostatic spray capsule 4 is mounted. The electrostatic spray capsule 4 can be fixed by a force applied by the conducting mechanisms 12 and 14, in contact with the conducting mechanisms 12 and 14, and can be firmly fixed in the electrostatic spray capsule holder 6 with unexpected separation limited.

In the conducting mechanisms 12 and 14, both of the ground terminal conducting mechanism 12 and the high voltage applying terminal conducting mechanism 14 may be elastic conducting mechanisms, or only one of them may be an elastic conducting mechanism and the other one may be non-elastic conducting mechanism. The elastic conducting mechanism may be an elastic conducting mechanism that can at least partially elastically deform and the non-elastic conducting mechanism may be a conducting mechanism that does not generally elastically deform.

At least one of the ground terminal conducting mechanism 12 and the high voltage applying terminal conducting mechanism 14 may include the connection terminals 122 and 142 connected with the high voltage generator 8, the contact members 124 and 144 brought in contact with the terminals 46 and 48, when the electrostatic spray capsule 4 is mounted, and the elastic members 126 and 146 being in contact with the contact members 124 and 144 and elastically supporting the contact members 124 and 144 toward the terminals 46 and 48.

When both of the ground terminal conducting mechanism 12 and the high voltage applying terminal conducting mechanism 14 are elastic conducting mechanisms, the ground terminal conducting mechanism 12 may include the connection terminal 122 connected with the high voltage generator 8, the contact member 124 brought in contact with the ground terminal 46, when the electrostatic spray capsule 4 is mounted, and the elastic member 126 being in contact with the contact member 124 and elastically supporting the contact member 124 toward the ground terminal 46. Further, the high voltage applying terminal conducting mechanism 14 may include the connection terminal 142 connected with the high voltage generator 8, the contact member 144 brought in contact with the high voltage applying terminal 48, when the electrostatic spray capsule 4 is mounted, and the elastic member 146 being in contact with the contact member 144 and elastically supporting the contact member 144 toward the high voltage applying terminal 48.

In the ground terminal conducting mechanism 12 and the high voltage applying terminal conducting mechanism 14, when the ground terminal conducting mechanism 12 is an elastic conducting mechanism and the high voltage applying terminal conducting mechanism 14 is a non-elastic conducting mechanism, the ground terminal conducting mechanism 12 may include the connection terminal 122 connected with the high voltage generator 8, the contact member 124 brought in contact with the ground terminal 46, when the electrostatic spray capsule 4 is mounted, and the elastic member 126 being in contact with the contact member 124 and elastically supporting the contact member 124 toward the ground terminal 46. In contrast, the high voltage applying terminal conducting mechanism 14 may include a contact connection terminal (not shown) that is connected with the high voltage generator 8 and that is brought in contact with the high voltage applying terminal 48, when the electrostatic spray capsule 4 is mounted.

In the ground terminal conducting mechanism 12 and the high voltage applying terminal conducting mechanism 14, when the ground terminal conducting mechanism 12 is a non-elastic conducting mechanism and the high voltage applying terminal conducting mechanism 14 is an elastic conducting mechanism, the ground terminal conducting mechanism 12 may include a contact connection terminal (not shown) that is connected with the high voltage generator 8 and that is brought in contact with the ground terminal 46, when the electrostatic spray capsule 4 is mounted. On the contrary, the high voltage applying terminal conducting mechanism 14 may include the connection terminal 142 connected with the high voltage generator 8, the contact member 144 brought in contact with the high voltage applying terminal 48, when the electrostatic spray capsule 4 is mounted, and the elastic member 146 being in contact with the contact member 144 and elastically supporting the contact member 144 toward the high voltage applying terminal 48.

The electrostatic spray capsule module 2 may include an elastic holder 16 that elastically supports the electrostatic spray capsule 4, when the electrostatic spray capsule 4 is mounted. The elastic holder 16 can come in contact with the terminal of the electrostatic spray capsule 4 and elastically support the electrostatic spray capsule 4 by elastically deforming, when the electrostatic spray capsule 4 is mounted. The electrostatic spray capsule 4 can be fixed by a force applied by the elastic holder 16, in contact with the elastic holder 16. The elastic holder 16 may have a spring 162 in the electrostatic spray capsule holder 6 and balls 164 that are supported by the spring 162 and brought in contact with the electrostatic spray capsule 4, when the electrostatic spray capsule 4 is mounted. The balls 164 of the elastic holder 16 may be arranged to face the contact member 126 of the ground terminal conducting mechanism 12. The balls 164 of the elastic holder 16 may be disposed at positions where they can come in contact with the ground terminal 44 of the electrostatic spray capsule 4.

The ground terminal conducting mechanism 12, the high voltage applying terminal conducting mechanism 14, and the elastic holder 16 may be spaced from each other in the electrostatic spray capsule holder 6, and can fix the electrostatic spray capsule 4 by pressing electrostatic spray capsule 4 in different directions.

Hereinafter, an example when both of the ground terminal conducting mechanism 12 and the high voltage applying terminal conducting mechanism 14 are elastic conducting mechanisms is described.

The ground terminal conducting mechanism 12 may include the connection terminal 122 connected with the high voltage generator 8, the contact member 124 brought in contact with the ground terminal 46, when the electrostatic spray capsule 4 is mounted, and the elastic member 126 being in contact with the contact member 124 and elastically supporting the contact member 124 toward the ground terminal 46.

The high voltage applying terminal conducting mechanism 14 may include the connection terminal 142 connected with the high voltage generator 8, the contact member 144 brought in contact with the high voltage applying terminal 48, when the electrostatic spray capsule 4 is mounted, and the elastic member 146 being in contact with the contact member 144 and elastically supporting the contact member 144 toward the high voltage applying terminal 48.

One of the contact member 124 of the ground terminal conducting mechanism 12 and the contact member 144 of the high voltage applying terminal conducting mechanism 14 may be disposed to face the balls 164 of the elastic holder 16. One of the ground terminal conducting mechanism 12 and the high voltage applying terminal conducting mechanism 14, and the elastic holder 16 may press the electrostatic spray capsule 4 in opposite directions to each other and the electrostatic spray capsule 4 can be stably fixed between one of the ground terminal conducting mechanism 12 and the high voltage applying terminal conducting mechanism 14 and the elastic holder 16.

The electrostatic spray capsule 6 may include a bottom plate 62 in which the high voltage applying terminal conducting mechanism 14 is disposed, a first wall 64 that is formed on the bottom plate 62 and in which the ground terminal conducting mechanism 12 is disposed, and a second wall 66 that faces the first wall 64 and in which the elastic holder 16 is disposed.

A receiving groove 68 in which the lower portion of the electrostatic spray capsule 4 can be inserted and received may be formed on the bottom plate 62. A space 70 where the contact member 144 of the high voltage applying terminal conducting mechanism 14 is disposed and protrudes may be stepped from the receiving groove 68, over the bottom plate 62.

In the bottom plate 62, an elastic member receiving groove 75 where the elastic member 146 of the high voltage applying terminal conducting mechanism 14 can be received may be formed and the contact member 144 of the high voltage applying terminal conducting mechanism 14 can be at least partially received in the elastic member receiving groove 75.

Referring to FIGS. 8 and 9, one end 142A of the connection terminal 142 of the high voltage applying terminal conducting mechanism 14 may be positioned outside the electrostatic spray capsule holder 6 and connected with the high voltage generator 8 by a lead wire. The other end 142B of the connection terminal 142 of the high voltage applying terminal conducting mechanism 14 may be positioned in the electrostatic spray capsule holder 6. The other end 142B of the connection terminal 142 of the high voltage applying terminal conducting mechanism 14 may be positioned in the elastic member receiving groove 75 and may be in contact with one of the contact member 144 and the elastic member 146 of the high voltage applying terminal conducting mechanism 14. The connection terminal 142 of the high voltage applying terminal conducting mechanism 14 may be bent at least one time, so that it has a height difference between one end 142A and the other end 142B. The connection terminal 142 of the high voltage applying terminal conducting mechanism 14 may be formed in a plate shape and disposed horizontally in the electrostatic spray capsule holder 6. In the connection terminal 142 of the high voltage applying terminal conducting mechanism 14, one end 142A may face the outside of the bottom plate 62 and the other end 142B may face the space 70.

The connection terminal 142 of the high voltage applying terminal conducting mechanism 14, which is brought in contact with the high voltage applying terminal 48, when the electrostatic spray capsule 4 is mounted, and is separated from the high voltage applying terminal 48, when the electrostatic spray capsule 4 is separated, may be disposed movably in the electrostatic spray capsule holder 6. The contact member 144 of the high voltage applying terminal conducting mechanism 14 may be formed in a sphere or a polygon and the high voltage applying terminal 48 can come in contact with it by sliding, when the electrostatic spray capsule 4 is mounted. When the electrostatic spray capsule 4 is mounted, the contact member 144 of the high voltage applying terminal conducting mechanism 14 can be moved toward the elastic member 146 of the high voltage applying terminal conducting mechanism 14 by being pushed by the high voltage applying terminal 48 and can be inserted into the elastic member receiving groove 75 while elastically deforming the elastic member 146 of the high voltage applying terminal conducting mechanism 14. When the electrostatic spray capsule 4 is separated, the contact member 144 of the high voltage applying terminal conducting mechanism 14 can be moved in the opposite direction to the elastic member 146 of the high voltage applying terminal conducting mechanism 14 by the elastic member 146 of the high voltage applying terminal conducting mechanism 14 and can partially protrude from the space 70.

The elastic member 146 of the high voltage applying terminal conducting mechanism 14 can be elastically deformed by the contact member 144 of the high voltage applying terminal conducting mechanism 14, when it is in the elastic member receiving groove 75, and it can move the contact member 144 of the high voltage applying terminal conducting mechanism 14, using a restoring force. The elastic member 146 of the high voltage applying terminal conducting mechanism 14 may be implemented by various springs such as a coil spring or a leaf spring.

The outer side of the electrostatic spray capsule 4 may be rounded. In the electrostatic spray capsule holder 6, the surfaces 72 and 74 facing the electrostatic spray capsule 4 may be rounded, similar to the outer side of the electrostatic spray capsule 4. In the bottom plate 6, an elastic member receiving groove 76 where the elastic member 126 of the ground terminal conducting mechanism 12 can be received may be formed and the contact member 124 of the ground terminal conducting mechanism 12 can be at least partially received in the elastic member receiving groove 76.

Referring to FIGS. 6 and 7, one end 122A of the connection terminal 122 of the ground terminal conducting mechanism 12 may be positioned outside the electrostatic spray capsule holder 6 and connected with the high voltage generator 8 by a lead wire. The other end 122B of the connection terminal 122 of the ground terminal conducting mechanism 12 may be positioned in the electrostatic spray capsule holder 6. The other end 122B of the connection terminal 122 of the ground terminal conducting mechanism 12 may be positioned in the elastic member receiving groove 76 and may be in contact with one of the contact member 124 and the elastic member 126 of the ground terminal conducting mechanism 12. The connection terminal 122 of the ground terminal conducting mechanism 12 may be bent at least one time, so that it has a height difference between one end 122A and the other end 122B. One end 122A of the connection terminal 122 of the ground terminal conducting mechanism 12 may be horizontally position over the electrostatic spray capsule holder 6 and the other end 122B may be horizontally positioned under the electrostatic spray capsule holder 6. One end 122A and the other end 122B of the connection terminal 122 of the ground terminal conducting mechanism 12 may be arranged in different directions. One end 122A of the connection terminal 122 of the ground terminal conducting mechanism 12 may face the outside of the electrostatic spray capsule holder 6 and the other end 122B may face the inside of the electrostatic spray capsule holder 6.

The contact member 124 of the ground terminal conducting mechanism 12, which comes in contact with the ground terminal 46, when the electrostatic spray capsule 4 is mounted, and is separated from the ground terminal 46, when the electrostatic spray capsule 4 is separated, may be disposed movably in the electrostatic spray capsule holder 6. The contact member 124 of the ground terminal conducting mechanism 12 may be formed in a sphere or a polygon and the ground terminal 46 can come in contact with it by sliding, when the electrostatic spray capsule 4 is mounted. When the electrostatic spray capsule 4 is mounted, the contact member 124 of the ground terminal conducting mechanism 12 can be moved toward the elastic member 126 of the ground terminal conducting mechanism 12 by being pushed by the ground terminal 46 and can be inserted into the elastic member receiving groove 76 while elastically deforming the elastic member 126 of the ground terminal conducting mechanism 12. When the electrostatic spray capsule 4 is separated, the contact member 124 of the ground terminal conducting mechanism 12 can be moved in the opposite direction to the elastic member 126 of the ground terminal conducting mechanism 12 by the elastic member 126 of the ground terminal conducting mechanism 12 and can partially protrude from the space 70.

The elastic member 126 of the ground terminal conducting mechanism 12 can be elastically deformed by the contact member 124 of the ground terminal conducting mechanism 12, when it is in the elastic member receiving groove 76, and it can move the contact member 124 of the ground terminal conducting mechanism 12, using a restoring force. The elastic member 126 of the ground terminal conducting mechanism 12 may be implemented by various springs such as a coil spring or a leaf spring.

The electrostatic spray capsule holder 6 may have a spring receiving groove 78 where the spring 162 of the elastic holder 16 can be received, and the balls 164 of the elastic holder 16 can be at least partially received in the spring receiving groove 78.

The spring 162 of the elastic holder 16 can be elastically deformed by the balls 164 of the elastic holder 16 and can move the balls 164 of the elastic holder 16, using a restoring force, in the spring receiving groove 78. The spring 163 of the elastic holder 16 may be implemented by various springs such as a coil spring or a leaf spring.

The balls 164 of the elastic holder 16, which are brought in contact with the ground terminal 46, when the electrostatic spray capsule 4 is mounted, and are separated from the ground terminal 46, when the electrostatic spray capsule 4 is separated, may be disposed movably in the electrostatic spray capsule holder 6. The balls 164 of the elastic holder 16 may be formed in a sphere or a polygon and the ground terminal 46 can come in contact with it by sliding, when the electrostatic spray capsule 4 is mounted. The balls 164 of the elastic holder 16 can be moved toward the spring 162 of the elastic holder 16 by being pushed by the ground terminal 46 and can be inserted into the spring receiving groove 78 while elastically deforming the spring 162 of the elastic holder 16, when the electrostatic spray capsule 4 is mounted. The balls 164 of the elastic holder 16 can be moved in the opposite direction to the sprig 162 of the elastic holder 16 by the spring 162 of the elastic holder 16 and can protrude from the electrostatic spray capsule holder 6, when the electrostatic spray capsule 4 is separated.

The contact member 124 of the ground terminal conducting mechanism 12 and the contact member 144 of the high voltage applying terminal conducting mechanism 14 may be disposed at different heights. The contact member 124 of the ground terminal conducting mechanism 12 and the contact member 144 of the high voltage applying terminal conducting mechanism 14 can press together portions at different heights of the electrostatic spray capsule 4.

The connection terminal 122 of the ground terminal conducting mechanism 12 and the connection terminal 142 of the high voltage applying terminal conducting mechanism 14 may protrude in different directions from the electrostatic spray capsule holder 6.

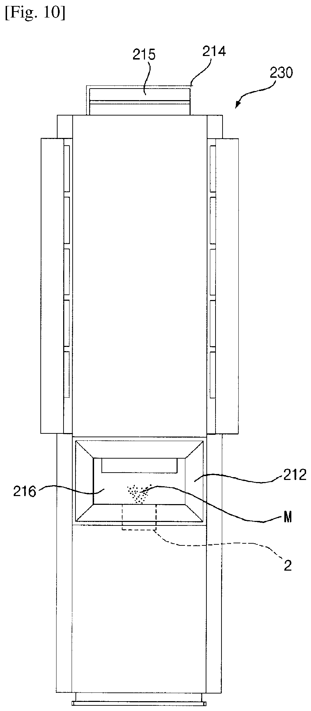

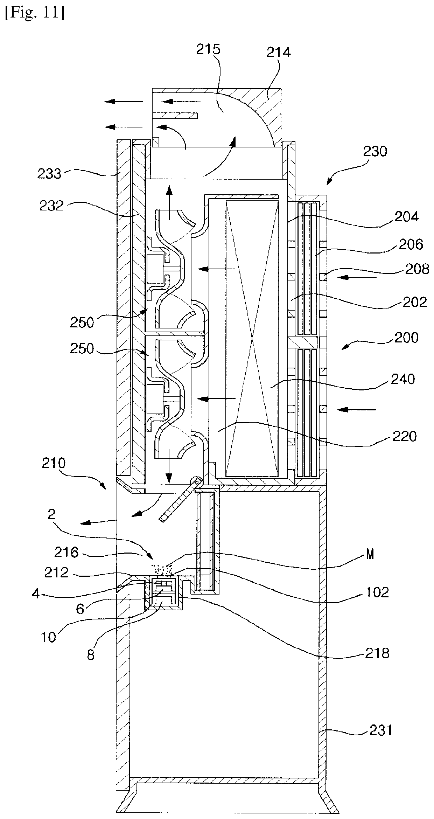

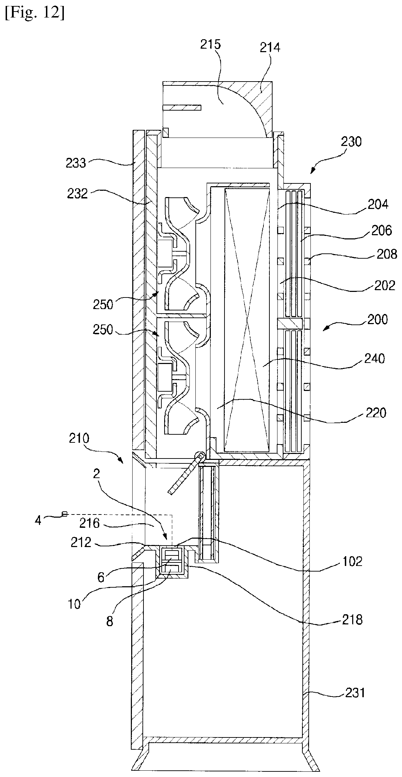

FIG. 10 is a front view of an embodiment of an air conditioner equipped with an electrostatic spray module according to an embodiment of the present invention, FIG. 11 is a cross-sectional view of an example of an air conditioner equipped with an electrostatic spray module according to an embodiment of the present invention, and FIG. 12 is a cross-sectional view when an electrostatic spray capsule is separated in an embodiment of an air conditioner equipped with an electrostatic spray module according to the present invention.

An air conditioner equipped with an electrostatic spray module may include: a body 230 that has an air intake unit 200, an air discharge unit 210, and an air channel 220 between the air intake unit 200 and the air discharge unit 210; a heat exchanger 240 disposed in the air channel 220; and a blowing unit 250 that takes air from the air intake unit 200 and sends the air to the air discharge unit 210 through the heat exchanger 240, in which the electrostatic spray module 2 may be disposed in the body 230.

The air intake unit 200 may include an intake body 204 having an air intake port 202. The air intake unit 200 may further include a filter that purifies the air sucked through the air intake port 202. The air intake unit 200 may further include a filter frame 208 with the filter 206 mounted. The filter frame 208 may be attached/detached to/from the intake body 204.

One or a plurality of air discharge units 210 may be disposed in the body 230. The air discharge unit 210 may include a discharge body 212 which is fixed and through which air is discharged to the interior. The air discharge unit 210 may further include a moving body 214 that is movably disposed.

The body 230 may include a base 231 supporting the intake body 204. The main body 230 may further include an air guide 232 that is equipped with the blowing unit 250 and guides the air passing through the heat exchanger 240. The body 230 may further include a front panel 233 disposed ahead of the air guide 232.

The discharge body 212 may have an air discharge space 216 that is open to discharge air to the interior. The discharge body 212 may have an air intake side through which the air sent from the blowing unit 250 flows inside. The discharge body 212 may have a discharge side through which the air flowing in the air discharge space 216 and mist M sprayed from the electrostatic spray module 2 are discharged together. The air discharge space 216 of the discharge body 212 may be sized such that a user can put his/her hand in/out through it. The front of the air discharge space 216 may be open and the air exchanging heat through the heat exchanger 240 and the mist M sprayed from the electrostatic spray module 2 can be discharged together forward from the discharge body 212. The discharge body 212 may have a module seat 218 in which the electrostatic spray module 2 can be received. The module seat 218 can receive the entire electrostatic spray module 2 or may receive a portion of the electrostatic spray module 2.

The moving body 214 may have a discharge channel 215 through which air is discharged. The moving body 214 can move up between the intake body 204 and the air guide 232 or move down between the intake body 204 and the air guide 232.

The heat exchanger 240 is disposed between the air intake unit 200 and the air discharge unit 210 and can allow the air sucked in the air intake unit 200 to exchange heat with a refrigerant.

The blowing unit 250 may be disposed opposite the heat exchanger 240. The blowing unit 250 may include a driving source such as a motor and a blower rotated by the driving source.

The electrostatic spray module 2 may be disposed in at least one of the discharge body 212 and the moving body 214 and the mist M sprayed from the electrostatic spray capsule 4 can be discharged far together with the air sent from the blowing unit 250. In the electrostatic spray module 2, the electrostatic spray capsule 4 itself may be replaced or the functional substance in the electrostatic spray capsule 4 may be replaced, and it is preferable that the electrostatic spray module 2 is installed at a position where the electrostatic spray capsule 4 can be conveniently attached/detached without disassembling the air conditioner. It is preferable that the electrostatic spray module 2 is installed at one of the discharge body 212 and the moving body 214 where service of the electrostatic spray capsule 4 is easy.

The electrostatic spray module 2 is disposed in the discharge body 212 and can spray a functional substance to the air discharge space 216. The electrostatic spray capsule 4 can be put into/out of the entrance 102 of the electrostatic spray module 2 through the air discharge space 216.

Hereinafter, the operation of the air conditioner equipped with the electrostatic spray module is described.

When the air conditioner is started, the high voltage generator 8 can apply high voltage to the conducting mechanisms 12 and 13 and the blowing unit 250 can be started.

The high voltage generated by the high voltage generator 8 can be applied to the electrostatic spray capsule 4 through the conducting mechanisms 12 and 14 and the electrostatic spray capsule 4 can spray the mist M, using an electric field generated therein. The mist M sprayed from the electrostatic spray capsule 4 can flow to the air discharge space 216 of the discharge body 214 through the entrance 102 of the housing 10.

When the blower 250 is started, the indoor air can flow to the heat exchanger 240 through the air intake unit 200 and pass through the heat exchanger 240. The air passing through the heat exchanger 240 can flow into the air discharge space 216 by the blowing unit 250. The air flowing in the air discharge space 216 can be mixed with the mist M in the air discharge space 216 and then discharged forward in the air discharge space 216. The air mixed with the mist M and discharged from the air discharge space 216 can spread the atomized functional substance to the interior while widely spreading in the interior.

On the other hand, a user may want to replace the functional substance A to be spread into the interior or additionally put in a functional substance A. The user can put out the electrostatic spray capsule 4 in the body 230 sequentially through the entrance 102 of the housing 10 and the air discharge space 216. The user can put another electrostatic spray capsule, which contains another functional substance and has the same structure as the electrostatic spray capsule 4 in the air conditioner, into the air discharge space 216 and can put it on the electrostatic spray capsule holder 6 through the entrance 102 of the housing 10, in the air discharge space 216. The user can add a functional substance into the electrostatic spray capsule 4 and put it in the air discharge space 216 and can put it on the electrostatic spray capsule holder 6 through the entrance 102 of the housing 10, in the air discharge space 216. When mounted on the electrostatic spray capsule holder 6, the electrostatic spray capsule 4 can be elastically fixed in contact with the conducting mechanisms 12 and 14 and the elastic holder 16. The electrostatic spray capsule 4 can be electrically connected with the high voltage generator 8 in contact with the conducting mechanisms 12 and 16.

## Claims

1. An electrostatic spray module comprising:
an electrostatic spray capsule (4) with a terminal (46, 48);
a high voltage generator (8);
an electrostatic spray capsule holder (6) where the electrostatic spray capsule (4) is attached/detached; and
a conducting mechanism (12, 14) mounted on the electrostatic spray capsule holder (6), connected with the high voltage generator (8), and coming in contact with the terminal (46, 48) by partially elastically deforming, when the electrostatic spray capsule (4) is mounted, wherein the conducting mechanism (12, 14) includes:
a connection terminal (48) connected with the high voltage generator (8);
a contact member (124) coming in contact with the terminal (48), when the electrostatic spray capsule (4) is mounted; and
an elastic member (126) elastically supporting the contact member (124) toward the terminal (48),
wherein the electrostatic spray module (2) further comprises:
a spring (162) disposed in the electrostatic spray capsule holder (6); and
an elastic holder (16) having balls (164) that are supported by the spring (162) and brought in contact with the electrostatic spray capsule (4), when the electrostatic spray capsule (4) is mounted.

2. The electrostatic spray module of claim 1, wherein the connection terminal (48) is connected with the elastic member (126).

3. The electrostatic spray module of claim 1, wherein the contact member (124) comes in contact with the connection terminal (48), when the electrostatic spray capsule (4) is mounted.

4. The electrostatic spray module of claim 1, wherein the terminal includes a ground terminal (46) with a portion exposed to the outside of the electrostatic spray capsule (4) and a high voltage applying terminal (48) with a portion exposed to the outside of the electrostatic spray capsule (4), and
the conducting mechanism includes a ground terminal conducting mechanism (12) electrically connected with the ground terminal (46) and a high voltage applying terminal conducting mechanism (14) electrically connected with the high voltage applying terminal (48).

5. The electrostatic spray module of claim 4, wherein one of a contact member of the ground terminal conducting mechanism (12) and a contact member of the high voltage applying terminal conducting mechanism (14) is disposed to face the balls (164).

6. An air conditioner comprising:
a body (230) that has an air intake unit (200), an air discharge unit (220), and an air channel (210) between the air intake unit (200) and the air discharge unit (220);
a heat exchanger (240) disposed in the air channel (210);
a blowing unit (250) that takes air from the air intake unit (200) and sends the air to the air discharge unit (220) through the heat exchanger (240); and
an electrostatic spray module (2) disposed in the body (230),
wherein the electrostatic spray module (2) is configured in accordance with one or more of the claims 1 to 5.

7. The air conditioner of claim 6, wherein the electrostatic spray module (2) includes a housing (10) having the electrostatic spray capsule holder (6) and an entrance (102) at a side through which the electrostatic spray capsule (4) is put inside/outside.

8. The air conditioner of claim 7, wherein the high voltage generator (8) is disposed in the housing (10).

9. The air conditioner of claim 6, wherein the air discharge unit (220) includes a discharge body (212) having an open air discharge space through which air is discharge to an interior,
the discharge body (212) has a module seat (281) in which the electrostatic spray module (4) is received, and
the electrostatic spray module (2) sprays a functional substance to the air discharge space.

## Patentansprüche

1. Elektrostatische Sprüheinheit, umfassend:
eine elektrostatische Sprühkapsel (4) mit einem Anschluss (46, 48);
einen Hochspannungsgenerator (8);
einen elektrostatischen Sprühkapselhalter (6), an dem die elektrostatische Sprühkapsel (4) angebracht/abgenommen wird; und
einen leitenden Mechanismus (12, 14), der an dem elektrostatischen Sprühkapselhalter (6) montiert ist, mit dem Hochspannungsgenerator (8) verbunden ist, und durch teilweises elastisches Verformen mit dem Anschluss (46, 48) in Kontakt kommt, wenn die elektrostatische Sprühstrahlkapsel (4) montiert wird, wobei der leitende Mechanismus (12, 14) aufweist:
einen Verbindungsanschluss (48), der mit dem Hochspannungsgenerator (8) verbunden ist;
ein Kontaktelement (124), das mit dem Anschluss (48) in Kontakt kommt, wenn die elektrostatische Sprühkapsel (4) montiert wird; und
ein elastisches Element (126), das das Kontaktelement (124) in der Richtung zu dem Anschluss (48) hin elastisch abstützt,
wobei die elektrostatische Sprüheinheit (2) ferner umfasst:
eine Feder (162), die in dem elektrostatischen Sprühkapselhalter (6) angeordnet ist; und
einen elastischen Halter (16) mit Kugeln (164), die durch die Feder (162) gestützt werden und in Kontakt mit der elektrostatischen Sprühkapsel (4) gebracht werden, wenn die elektrostatische Sprühkapsel (4) montiert wird.

2. Elektrostatische Sprüheinheit nach Anspruch 1, wobei der Verbindungsanschluss (48) mit dem elastischen Element (126) verbunden ist.

3. Elektrostatische Sprüheinheit nach Anspruch 1, wobei das Kontaktelement (124) mit dem Verbindungsanschluss (48) in Kontakt kommt, wenn die elektrostatische Sprühkapsel (4) montiert wird.

4. Elektrostatische Sprüheinheit nach Anspruch 1, wobei der Anschluss einen Erdungsanschluss (46) mit einem Abschnitt, der zur Außenseite der elektrostatischen Sprühkapsel (4) freiliegt, und einen Hochspannungsanlegeanschluss (48) mit einem Bereich, der zur Außenseite der elektrostatischen Sprühkapsel (4) freiliegt, aufweist, und
der leitende Mechanismus einen leitenden Erdungsanschluss-Mechanismus (12), der elektrisch mit dem Erdungsanschluss (46) verbunden ist, und einen leitenden Hochspannungsanlegeanschluss-Mechanismus (14), der elektrisch mit dem Hochspannungsanlegeanschluss (48) verbunden ist, aufweist.

5. Elektrostatische Sprüheinheit nach Anspruch 4, wobei eines von einem Kontaktelement des leitenden Erdungsanschluss-Mechanismus (12) und einem Kontaktelement des leitenden Hochspannungsanlegeanschluss-Mechanismus (14) den Kugeln (164) zugewandt angeordnet ist.

6. Klimaanlage, umfassend:
einen Körper (230), der eine Lufteinlasseinheit (200), eine Luftausspeiseeinheit (220), und einen Luftkanal (210) zwischen der Lufteinlasseinheit (200) und der Luftausspeiseeinheit (220) aufweist;
einen Wärmetauscher (240), der in dem Luftkanal (210) angeordnet ist;
eine Gebläseeinheit (250), die Luft von der Lufteinlasseinheit (200) aufnimmt und die Luft durch den Wärmetauscher (240) zu der Luftausspeiseeinheit (220) sendet; und
eine elektrostatische Sprüheinheit (2), die in dem Körper (230) angeordnet ist,
wobei die elektrostatische Sprüheinheit (2) gemäß einem oder mehreren der Ansprüche 1 bis 5 ausgebildet ist.

7. Klimaanlage nach Anspruch 6, wobei die elektrostatische Sprüheinheit (2) ein Gehäuse (10) mit dem elektrostatischen Sprühkapselhalter (6) und einem Eingang (102) an einer Seite, durch den die elektrostatische Sprühkapsel (4) hinein/hinaus getan wird, aufweist.

8. Klimaanlage nach Anspruch 7, wobei der Hochspannungsgenerator (8) in dem Gehäuse (10) angeordnet ist.

9. Klimaanlage nach Anspruch 6, wobei die Luftausspeiseeinheit (220) einen Ausspeisekörper (212) mit einem offenen Luftausspeiseraum aufweist, durch den Luft zu einem Innenraum ausgespeist wird,
wobei der der Ausspeisekörper (212) einen Modulsitz (281) aufweist, in dem die elektrostatische Sprüheinheit (4) aufgenommen ist, und
die elektrostatische Sprüheinheit (2) eine funktionelle Substanz in den Luftausspeiseraum sprüht.

## Revendications

1. Module de pulvérisation électrostatique comprenant :
une capsule de pulvérisation électrostatique (4) avec une borne (46,48) ;
un générateur de haute tension (8) ;
un support de capsule de pulvérisation électrostatique (6) où la capsule de pulvérisation électrostatique est fixée/détachée ; et
un mécanisme de conduction (12,14) monté sur le support de capsule de pulvérisation électrostatique (6), connecté avec le générateur de haute tension (8) et venant en contact avec la borne (46,48) par déformation élastique partielle, lorsque la capsule de pulvérisation électrostatique (4) est montée, dans lequel le mécanisme de conduction (12,14)inclut :
une borne de connexion (48) connectée au générateur de haute tension (8) ;
un élément de contact (124) venant en contact avec la borne (48), lorsque la capsule de pulvérisation électrostatique (4) est montée ; et
un élément élastique (126) soutenant élastiquement l'élément de contact (124) dans la direction de la borne (48),
dans lequel le module de pulvérisation électrostatique comprend en outre :
un ressort (162) disposé dans le support de capsule de pulvérisation électrostatique (6) ; et
un support élastique (16) ayant des billes (164) qui sont soutenues par le ressort (162) et amenées en contact avec la capsule de pulvérisation électrostatique (4), lorsque la capsule de pulvérisation électrostatique (4) est montée.

2. Module de pulvérisation électrostatique selon la revendication 1, dans lequel la borne de connexion (48) est connectée avec l'élément élastique (126).

3. Module de pulvérisation électrostatique selon la revendication 1, dans lequel l'élément de contact (124) vient en contact avec la borne de connexion (48), lorsque la capsule de pulvérisation électrostatique (4) est montée.

4. Module de pulvérisation électrostatique selon la revendication 1, dans lequel la borne inclut une borne de masse (46) avec une portion exposée à l'extérieur de la capsule de pulvérisation électrostatique (4) et une borne d'application de haute tension (48) avec une portion exposée à l'extérieur de la capsule de pulvérisation électrostatique (4), et
le mécanisme de conduction inclut un mécanisme de conduction de borne de masse (12) connecté électriquement à la borne de masse (46) et un mécanisme de conduction de borne d'application de haute tension (14) connecté électriquement à la borne d'application de haute tension (48).

5. Module de pulvérisation électrostatique selon la revendication 4, dans lequel un d'un élément de contact du mécanisme de conduction de borne de masse (12) et un élément de contact du mécanisme de conduction de borne d'application de haute tension (14) est disposé afin de faire face aux billes (164).

6. Appareil de conditionnement d'air comprenant :
un corps (230) qui possède une unité d'admission d'air (200), une unité d'évacuation d'air (220) et un canal d'air (210) entre l'unité d'admission d'air (200) et l' unité d'évacuation d'air (220) ;
un échangeur thermique (240) disposé dans le canal d'air (210) ;
une unité de soufflage (250) qui prélève l'air provenant de l'unité d'admission d'air (200) et envoie l'air vers l'unité d'évacuation d'air (220) à travers l'échangeur thermique (240) ; et
un module de pulvérisation électrostatique (2) disposé dans le corps (230),
dans lequel le module de pulvérisation électrostatique (2) est configuré conformément à une ou plusieurs des revendications 1 à 5.

7. Appareil de conditionnement d'air selon la revendication 6, dans lequel le module de pulvérisation électrostatique (2) inclut un logement (10) ayant le support de capsule de pulvérisation électrostatique (6) et une entrée (102) au niveau d'un côté à travers lequel la capsule de pulvérisation électrostatique (4) est placée à l'intérieur/l'extérieur.

8. Appareil de conditionnement d'air selon la revendication 7, dans lequel le générateur de haute tension (8) est disposé dans le logement (10).

9. Appareil de conditionnement d'air selon la revendication 6, dans lequel l'unité d'évacuation d'air (220) inclut un corps de décharge (212) ayant un espace d'évacuation d'air ouvert à travers lequel l'air est évacué vers un intérieur,
le corps d'évacuation (212) possède un siège de module (281) dans lequel le module de pulvérisation électrostatique (4) est reçu, et
le module de pulvérisation électrostatique (2) pulvérise une substance fonctionnelle dans l'espace d'évacuation d'air.
